# EUROPÄISCHE PATENTANMELDUNG

(11) **EP 1 106 695 A2**
(43) Veröffentlichungstag der Anmeldung: **13.06.2001**
(21) Anmeldenummer: 00710033.2
(22) Anmeldetag: 15.11.2000
(51) Int. Cl.: C12N 15/54, C12N 9/10, C12P 13/04, C12P 13/08

(54) **Verfahren zur fermentativen Herstellung von L-Aminosäuren unter Verwendung coryneformer Bakterien in denen man das glyA-Gen abschwächt**

(30) Priorität: 09.12.1999 DE 19959329
(71) Anmelder: Degussa-Hüls Aktiengesellschaft, 60287 Frankfurt am Main (DE); FORSCHUNGSZENTRUM JÜLICH GMBH, 52425 Jülich (DE)
(72) Erfinder: Ziegler, Petra, 52062 Aachen (DE); Eggeling, Lothar, Dr., 52428 Jülich (DE); Sahm, Hermann, Prof., 52428 Jülich (DE); Thierbach, Georg, Dr., 33613 Bielefeld (DE); Pfefferle, Walter, Dr., 33790 Halle (Westf.) (DE)

(57) **Zusammenfassung**

Die Erfindung betrifft ein Verfahren zur Herstellung von L-Aminosäuren, bei dem man folgende Schritte durchführt, 7.
a) Fermentation der die gewünschte L-Aminosäure produzierenden Bakterien, in denen man zumindest das glyA-Gen, insbesondere durch Entfernung des natürlichen Promoters abschwächt,
b) Anreicherung des gewünschten Produkts im Medium oder in den Zellen der Bakterien und
c) Isolieren der L-Aminosäure,
und gegebenenfalls Bakterien einsetzt, in denen man zusätzlich weitere Gene des Biosyntheseweges der gewünschten L-Aminosäure verstärkt, oder Bakterien einsetzt, in denen die Stoffwechselwege zumindest teilweise ausgeschaltet sind, die die Bildung der gewünschten L-Aminosäure verringern, und Nukleotidsequenzen der lacI-tac-5`glyA bzw. lacI-tac-glyA-Einheit.

SEQUENZPROTOKOLL

<110> Degussa-Hüls AG
      Forschungszentrum Jülich GmbH

<120> Verfahren zur fermentativen Herstellung von
      L-Aminosäuren unter Verwendung coryneformer Bakterien.

<130> 990200 BT

<140>
<141>

<160> 4

<170> PatentIn Ver. 2.1

<210> 1
<211> 438
<212> DNA
<213> Corynebacterium glutamicum ATCC13032

<220>
<221> N_region
<222> (1)..(438)
<223> 5'glyA

<400> 1
atgaccgatg cccaccaagc ggacgatgtc cgttaccagc cactgaacga gcttgatcct 60
gaggtggctg ctgccatcgc tggggaactt gcccgtcaac gcgatacatt agagatgatc 120
gcgtctgaga acttcgttcc ccgttctgtt ttgcaggcgc agggttctgt tcttaccaat 180
aagtatgccg agggttaccc tggccgccgt tactacggtg gttgcgaaca agttgacatc 240
attgaggatc ttgcacgtga tcgtgcgaag gctctcttcg gtgcagagtt cgccaatgtt 300
cagcctcact ctggcgcaca ggctaatgct gctgtgctga tgactttggc tgagccaggc 360
gacaagatca tgggtctgtc tttggctcat ggtggtcact tgacccacgg aatgaagttg 420
aacttctccg gaaagctg                                                438


<210> 2
<211> 2000
<212> DNA
<213> Künstliche Sequenz

<220>
<223> Beschreibung der künstlichen Sequenz:
      lacI-tac-5'glyA

<220>
<221> gene
<222> Complement((6)..(1097))
<223> lacI

<220>
<221> promoter
<222> (1391)..(1434)
<223> tac

<220>
<221> N_region

<222> (1562)..(1999)
<223> 5'glyA

<400> 2
aattctcact gcccgctttc cagtcgggaa acctgtcgtg ccagctgcat taatgaatcg 60
gccaacgcgc ggggagaggc ggtttgcgta ttgggcgcca gggtggtttt tcttttcacc 120
agtgagacgg gcaacagctg attgcccttc accgcctggc cctgagagag ttgcagcaag 180
cggtccacgc tggtttgccc cagcaggcga aaatcctgtt tgatggtggt tgacggcggg 240
atataacatg agctgtcttc ggtatcgtcg tatcccacta ccgagatatc cgcaccaacg 300
cgcagcccgg actcggtaat ggcgcgcatt gcgcccagcg ccatctgatc gttggcaacc 360
agcatcgcag tgggaacgat gccctcattc agcatttgca tggtttgttg aaaaccggac 420
atggcactcc agtcgccttc ccgttccgct atcggctgaa tttgattgcg agtgagatat 480
ttatgccagc cagccagacg cagacgcgcc gagacagaac ttaatgggcc cgctaacagc 540
gcgatttgct ggtgacccaa tgcgaccaga tgctccacgc ccagtcgcgt accgtcttca 600
tgggagaaaa taatactgtt gatgggtgtc tggtcagaga catcaagaaa taacgccgga 660
acattagtgc aggcagcttc cacagcaatg gcatcctggt catccagcgg atagttaatg 720
atcagcccac tgacgcgttg cgcgagaaga ttgtgcaccg ccgctttaca ggcttcgacg 780
ccgcttcgtt ctaccatcga caccaccacg ctggcaccca gttgatcggc gcgagattta 840
atcgccgcga caatttgcga cggcgcgtgc agggccagac tggaggtggc aacgccaatc 900
agcaacgact gtttgcccgc cagttgttgt gccacgcggt tgggaatgta attcagctcc 960
gccatcgccg cttccacttt ttcccgcgtt ttcgcagaaa cgtggctggc ctggttcacc 1020
acgcgggaaa cggtctgata agagacaccg gcatactctg cgacatcgta taacgttact 1080
ggtttcacat tcaccaccct gaattgactc tcttccgggc gctatcatgc cataccgcga 1140
aaggttttgc accattcgat ggtgtcaacg taaatgcatg ccgcttcgcc ttcgcgcgcg 1200
aattgcaagc tgatccgggc ttatcgactg cacggtgcac caatgcttct ggcgtcaggc 1260
agccatcgga agctgtggta tggctgtgca ggtcgtaaat cactgcataa ttcgtgtcgc 1320
tcaaggcgca ctcccgttct ggataatgtt ttttgcgccg acatcataac ggttctggca 1380
aatattctga aatgagctgt tgacaattaa tcatcggctc gtataatgtg tggaattgtg 1440
agcggataac aatttcacac aggaaacaga attaaaagat atgaccatga ttacgccaag 1500
cttgcatgcc tgcaggtcga ctctagagga tcattcgtct tgtgaaaggt tagctgacct 1560
gatgaccgat gcccaccaag cggacgatgt ccgttaccag ccactgaacg agcttgatcc 1620
tgaggtggct gctgccatcg ctggggaact tgcccgtcaa cgcgatacat tagagatgat 1680
cgcgtctgag aacttcgttc cccgttctgt tttgcaggcg cagggttctg ttcttaccaa 1740
taagtatgcc gagggttacc ctggccgccg ttactacggt ggttgcgaac aagttgacat 1800
cattgaggat cttgcacgtg atcgtgcgaa ggctctcttc ggtgcagagt tcgccaatgt 1860
tcagcctcac tctggcgcac aggctaatgc tgctgtgctg atgactttgg ctgagccagg 1920
cgacaagatc atgggtctgt ctttggctca tggtggtcac ttgacccacg gaatgaagtt 1980
gaacttctcc ggaaagctgg 2000


<210> 3
<211> 2866
<212> DNA
<213> Künstliche Sequenz

<220>
<223> Beschreibung der künstlichen Sequenz:lacI-tac-glyA

<220>
<221> gene
<222> Complement((6)..(1097))
<223> lacI

<220>
<221> promoter
<222> (1391)..(1434)
<223> tac

<220>

<221> CDS
<222> (1562)..(2863)
<223> glyA

<400> 3
aattctcact gcccgctttc cagtcgggaa acctgtcgtg ccagctgcat taatgaatcg 60

gccaacgcgc ggggagaggc ggtttgcgta ttgggcgcca gggtggtttt tcttttcacc 120

agtgagacgg gcaacagctg attgcccttc accgcctggc cctgagagag ttgcagcaag 180

cggtccacgc tggtttgccc cagcaggcga aaatcctgtt tgatggtggt tgacggcggg 240

atataacatg agctgtcttc ggtatcgtcg tatcccacta ccgagatatc cgcaccaacg 300

cgcagcccgg actcggtaat ggcgcgcatt gcgcccagcg ccatctgatc gttggcaacc 360

agcatcgcag tgggaacgat gccctcattc agcatttgca tggtttgttg aaaaccggac 420

atggcactcc agtcgccttc ccgttccgct atcggctgaa tttgattgcg agtgagatat 480

ttatgccagc cagccagacg cagacgcgcc gagacagaac ttaatgggcc cgctaacagc 540

gcgatttgct ggtgacccaa tgcgaccaga tgctccacgc ccagtcgcgt accgtcttca 600

tgggagaaaa taatactgtt gatgggtgtc tggtcagaga catcaagaaa taacgccgga 660

acattagtgc aggcagcttc cacagcaatg gcatcctggt catccagcgg atagttaatg 720

atcagcccac tgacgcgttg cgcgagaaga ttgtgcaccg ccgctttaca ggcttcgacg 780

ccgcttcgtt ctaccatcga caccaccacg ctggcaccca gttgatcggc gcgagattta 840

atcgccgcga caatttgcga cggcgcgtgc agggccagac tggaggtggc aacgccaatc 900

agcaacgact gtttgcccgc cagttgttgt gccacgcggt tgggaatgta attcagctcc 960

gccatcgccg cttccacttt ttcccgcgtt ttcgcagaaa cgtggctggc ctggttcacc 1020

acgcgggaaa cggtctgata agagacaccg gcatactctg cgacatcgta taacgttact 1080

ggtttcacat tcaccaccct gaattgactc tcttccgggc gctatcatgc cataccgcga 1140

aaggttttgc accattcgat ggtgtcaacg taaatgcatg ccgcttcgcc ttcgcgcgcg 1200

aattgcaagc tgatccgggc ttatcgactg cacggtgcac caatgcttct ggcgtcaggc 1260

agccatcgga agctgtggta tggctgtgca ggtcgtaaat cactgcataa ttcgtgtcgc 1320

tcaaggcgca ctcccgttct ggataatgtt ttttgcgccg acatcataac ggttctggca 1380

aatattctga aatgagctgt tgacaattaa tcatcggctc gtataatgtg tggaattgtg 1440

agcggataac aatttcacac aggaaacaga attaaaagat atgaccatga ttacgccaag 1500

cttgcatgcc tgcaggtcga ctctagagga tcattcgtct tgtgaaaggt tagctgacct 1560

g atg acc gat gcc cac caa gcg gac gat gtc cgt tac cag cca ctg aac 1609
Met Thr Asp Ala His Gln Ala Asp Asp Val Arg Tyr Gln Pro Leu Asn
1               5                   10                  15

gag ctt gat cct gag gtg gct gct gcc atc gct ggg gaa ctt gcc cgt 1657
Glu Leu Asp Pro Glu Val Ala Ala Ala Ile Ala Gly Glu Leu Ala Arg
            20                  25                  30

caa cgc gat aca tta gag atg atc gcg tct gag aac ttc gtt ccc cgt 1705
Gln Arg Asp Thr Leu Glu Met Ile Ala Ser Glu Asn Phe Val Pro Arg
        35                  40                  45

tct gtt ttg cag gcg cag ggt tct gtt ctt acc aat aag tat gcc gag 1753
Ser Val Leu Gln Ala Gln Gly Ser Val Leu Thr Asn Lys Tyr Ala Glu
    50                  55                  60

ggt tac cct ggc cgc cgt tac tac ggt ggt tgc gaa caa gtt gac atc 1801
Gly Tyr Pro Gly Arg Arg Tyr Tyr Gly Gly Cys Glu Gln Val Asp Ile
65                  70                  75                  80

att gag gat ctt gca cgt gat cgt gcg aag gct ctc ttc ggt gca gag 1849
Ile Glu Asp Leu Ala Arg Asp Arg Ala Lys Ala Leu Phe Gly Ala Glu
                85                  90                  95

ttc gcc aat gtt cag cct cac tct ggc gca cag gct aat gct gct gtg 1897
Phe Ala Asn Val Gln Pro His Ser Gly Ala Gln Ala Asn Ala Ala Val
            100                 105                 110

ctg atg act ttg gct gag cca ggc gac aag atc atg ggt ctg tct ttg 1945
Leu Met Thr Leu Ala Glu Pro Gly Asp Lys Ile Met Gly Leu Ser Leu
        115                 120                 125

gct cat ggt ggt cac ttg acc cac gga atg aag ttg aac ttc tcc gga 1993
Ala His Gly Gly His Leu Thr His Gly Met Lys Leu Asn Phe Ser Gly
    130                 135                 140

aag ctg tac gag gtt gtt gcg tac ggt gtt gat cct gag acc atg cgt 2041
Lys Leu Tyr Glu Val Val Ala Tyr Gly Val Asp Pro Glu Thr Met Arg
145                 150                 155                 160

gtt gat atg gat cag gtt cgt gag att gct ctg aag gag cag cca aag 2089
Val Asp Met Asp Gln Val Arg Glu Ile Ala Leu Lys Glu Gln Pro Lys
                165                 170                 175

gta att atc gct ggc tgg tct gca tac cct cgc cac ctt gat ttc gag 2137
Val Ile Ile Ala Gly Trp Ser Ala Tyr Pro Arg His Leu Asp Phe Glu
            180                 185                 190

gct ttc cag tct att gct gcg gaa gtt ggc gcg aag ctg tgg gtc gat 2185
Ala Phe Gln Ser Ile Ala Ala Glu Val Gly Ala Lys Leu Trp Val Asp
        195                 200                 205

atg gct cac ttc gct ggt ctt gtt gct gct ggt ttg cac cca agc cca 2233
Met Ala His Phe Ala Gly Leu Val Ala Ala Gly Leu His Pro Ser Pro
    210                 215                 220

gtt cct tac tct gat gtt gtt tct tcc act gtc cac aag act ttg ggt    2281
Val Pro Tyr Ser Asp Val Val Ser Ser Thr Val His Lys Thr Leu Gly
225                 230                 235                 240

gga cct cgt tcc ggc atc att ctg gct aag cag gag tac gcg aag aag    2329
Gly Pro Arg Ser Gly Ile Ile Leu Ala Lys Gln Glu Tyr Ala Lys Lys
                245                 250                 255

ctg aac tct tcc gta ttc cca ggt cag cag ggt ggt cct ttg atg cac    2377
Leu Asn Ser Ser Val Phe Pro Gly Gln Gln Gly Gly Pro Leu Met His
            260                 265                 270

gca gtt gct gcg aag gct act tct ttg aag att gct ggc act gag cag    2425
Ala Val Ala Ala Lys Ala Thr Ser Leu Lys Ile Ala Gly Thr Glu Gln
        275                 280                 285

ttc cgt gac cgt cag gct cgc acg ttg gag ggt gct cgc att ctt gct    2473
Phe Arg Asp Arg Gln Ala Arg Thr Leu Glu Gly Ala Arg Ile Leu Ala
    290                 295                 300

gag cgt ctg act gct tct gat gcg aag gcc gct ggc gtg gat gtc ttg    2521
Glu Arg Leu Thr Ala Ser Asp Ala Lys Ala Ala Gly Val Asp Val Leu
305                 310                 315                 320

acc ggt ggc act gat gtg cac ttg gtt ttg gct gat ctg cgt aac tcc    2569
Thr Gly Gly Thr Asp Val His Leu Val Leu Ala Asp Leu Arg Asn Ser
                325                 330                 335

cag atg gat ggc cag cag gcg gaa gat ctg ctg cac gag gtt ggt atc    2617
Gln Met Asp Gly Gln Gln Ala Glu Asp Leu Leu His Glu Val Gly Ile
            340                 345                 350

act gtg aac cgt aac gcg gtt cct ttc gat cct cgt cca cca atg gtt    2665
Thr Val Asn Arg Asn Ala Val Pro Phe Asp Pro Arg Pro Pro Met Val
        355                 360                 365

act tct ggt ctg cgt att ggt act cct gcg ctg gct acc cgt ggt ttc    2713
Thr Ser Gly Leu Arg Ile Gly Thr Pro Ala Leu Ala Thr Arg Gly Phe
    370                 375                 380

gat att cct gca ttc act gag gtt gca gac atc att ggt act gct ttg    2761
Asp Ile Pro Ala Phe Thr Glu Val Ala Asp Ile Ile Gly Thr Ala Leu
385                 390                 395                 400

gct aat ggt aag tcc gca gac att gag tct ctg cgt ggc cgt gta gca    2809
Ala Asn Gly Lys Ser Ala Asp Ile Glu Ser Leu Arg Gly Arg Val Ala
                405                 410                 415

aag ctt gct gca gat tac cca ctg tat gag ggc ttg gaa gac tgg acc    2857
Lys Leu Ala Ala Asp Tyr Pro Leu Tyr Glu Gly Leu Glu Asp Trp Thr
            420                 425                 430

atc gtc taa                                                        2866
Ile Val

<210> 4
<211> 434

<212> PRT

<400> 4
Met Thr Asp Ala His Gln Ala Asp Asp Val Arg Tyr Gln Pro Leu Asn
1               5                   10                  15

Glu Leu Asp Pro Glu Val Ala Ala Ala Ile Ala Gly Glu Leu Ala Arg
            20                  25                  30

Gln Arg Asp Thr Leu Glu Met Ile Ala Ser Glu Asn Phe Val Pro Arg
        35                  40                  45

Ser Val Leu Gln Ala Gln Gly Ser Val Leu Thr Asn Lys Tyr Ala Glu
    50                  55                  60

Gly Tyr Pro Gly Arg Arg Tyr Tyr Gly Gly Cys Glu Gln Val Asp Ile
65                  70                  75                  80

Ile Glu Asp Leu Ala Arg Asp Arg Ala Lys Ala Leu Phe Gly Ala Glu
                85                  90                  95

Phe Ala Asn Val Gln Pro His Ser Gly Ala Gln Ala Asn Ala Ala Val
            100                 105                 110

Leu Met Thr Leu Ala Glu Pro Gly Asp Lys Ile Met Gly Leu Ser Leu
        115                 120                 125

Ala His Gly Gly His Leu Thr His Gly Met Lys Leu Asn Phe Ser Gly
    130                 135                 140

Lys Leu Tyr Glu Val Val Ala Tyr Gly Val Asp Pro Glu Thr Met Arg
145                 150                 155                 160

Val Asp Met Asp Gln Val Arg Glu Ile Ala Leu Lys Glu Gln Pro Lys
                165                 170                 175

Val Ile Ile Ala Gly Trp Ser Ala Tyr Pro Arg His Leu Asp Phe Glu
            180                 185                 190

Ala Phe Gln Ser Ile Ala Ala Glu Val Gly Ala Lys Leu Trp Val Asp
        195                 200                 205

Met Ala His Phe Ala Gly Leu Val Ala Ala Gly Leu His Pro Ser Pro
    210                 215                 220

Val Pro Tyr Ser Asp Val Val Ser Ser Thr Val His Lys Thr Leu Gly
225                 230                 235                 240

Gly Pro Arg Ser Gly Ile Ile Leu Ala Lys Gln Glu Tyr Ala Lys Lys
                245                 250                 255

Leu Asn Ser Ser Val Phe Pro Gly Gln Gln Gly Gly Pro Leu Met His
            260                 265                 270

Ala Val Ala Ala Lys Ala Thr Ser Leu Lys Ile Ala Gly Thr Glu Gln
        275                 280                 285

Phe Arg Asp Arg Gln Ala Arg Thr Leu Glu Gly Ala Arg Ile Leu Ala
    290                 295                 300

Glu Arg Leu Thr Ala Ser Asp Ala Lys Ala Ala Gly Val Asp Val Leu
305                 310                 315                 320

Thr Gly Gly Thr Asp Val His Leu Val Leu Ala Asp Leu Arg Asn Ser
                325                 330                 335

Gln Met Asp Gly Gln Gln Ala Glu Asp Leu Leu His Glu Val Gly Ile
            340                 345                 350

Thr Val Asn Arg Asn Ala Val Pro Phe Asp Pro Arg Pro Pro Met Val
        355                 360                 365

Thr Ser Gly Leu Arg Ile Gly Thr Pro Ala Leu Ala Thr Arg Gly Phe
    370                 375                 380

Asp Ile Pro Ala Phe Thr Glu Val Ala Asp Ile Ile Gly Thr Ala Leu
385                 390                 395                 400

Ala Asn Gly Lys Ser Ala Asp Ile Glu Ser Leu Arg Gly Arg Val Ala
                405                 410                 415

Lys Leu Ala Ala Asp Tyr Pro Leu Tyr Glu Gly Leu Glu Asp Trp Thr
            420                 425                 430

Ile Val

## Beschreibung

Gegenstand der Erfindung ist ein Verfahren zur fermentativen Herstellung von L-Aminosäuren, insbesondere L-Threonin, unter Verwendung coryneformer Bakterien, in denen das glyA-Gen abgeschwächt ist.

### Stand der Technik

L-Aminosäuren finden in der Tierernährung, in der Humanmedizin und in der pharmazeutischen Industrie Anwendung.

Es ist bekannt, daß Aminosäuren durch Fermentation von Stämmen coryneformer Bakterien, insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen, wie z. B. Rührung und Versorgung mit Sauerstoff, oder die Zusammensetzung der Nährmedien, wie z. B. die Zuckerkonzentration während der Fermentation, oder die Aufarbeitung zur Produktform durch z. B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z. B. das Threonin-Analogon α-Amino-β-hydroxyvaleriansäure (AHV) oder auxotroph für regulatorisch bedeutsame Metabolite sind und L-Aminosäuren wie z. B. Threonin produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man einzelne Aminosäure-Biosynthesegene amplifiziert und die Auswirkung auf die L-Aminosäure-Produktion untersucht. Übersichtsartikel hierzu findet man unter anderem bei Kinoshita ("Glutamic Acid Bacteria,,, in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6, 261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich die Aufgabe gestellt, neue Grundlagen für verbesserte Verfahren zur fermentativen Herstellung von L-Aminosäuren mit coryneformen Bakterien bereitzustellen.

### Beschreibung der Erfindung

L-Aminosäuren finden in der Humanmedizin und in der pharmazeutischen Industrie, in der Lebensmittelindustrie und ganz besonders in der Tierernährung Anwendung. Es besteht daher ein allgemeines Interesse daran, neue verbesserte Verfahren zur Herstellung von Aminosäuren bereitzustellen.

Wenn im Folgenden L-Aminosäure erwähnt wird, ist damit L-Threonin oder L-Isoleucin gemeint.

Gegenstand der Erfindung ist ein Verfahren zur fermentativen Herstellung von L-Aminosäuren unter Verwendung von coryneformen Bakterien, in denen man zumindest die für das glyA-Genprodukt kodierende Nucleotidsequenz (glyA-Gen) abschwächt, insbesondere auf niedrigem Niveau exprimiert, das gewünschte Produkt im Medium oder in den Zellen anreichert und die L-Aminosäure isoliert.

Die eingesetzten Stämme produzieren bevorzugt bereits vor der Abschwächung des glyA-Gens L-Aminosäuren.

Bevorzugte Ausführungsformen finden sich in den Ansprüchen.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA (hier glyA-Gen) kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Die Mikroorganismen, die Gegenstand der vorliegenden Erfindung sind, können Aminosäuren aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme
wie beispielsweise die L-Threonin produzierenden Stämme
Corynebacterium glutamicum ATCC21649
Brevibacterium flavum BB69
Brevibacterium flavum DSM5399
Brevibacterium lactofermentum FERM-BP 269
Brevibacterium lactofermentum TBB-10 und
wie beispielsweise die L-Isoleucin produzierenden Stämme
Corynebacterium glutamicum ATCC 14309
Corynebacterium glutamicum ATCC 14310
Corynebacterium glutamicum ATCC 14311
Corynebacterium glutamicum ATCC 15168
Corynebacterium ammoniagenes ATCC 6871.

Es wurde gefunden, daß coryneforme Bakterien nach Abschwächung des glyA-Gens in verbesserter Weise L-Aminosäuren produzieren.

Das glyA-Gen codiert für das Enzym Serinhydroxymethyltransferase (EC 2.1.2.1). Die Nukleotidsequenz des glyA-Gens wurde in der japanischen Offenlegungsschrift JP-A-08107788 beschrieben. Das in der angegebenen Textstelle beschriebene glyA-Gen kann erfindungsgemäß verwendet werden. Weiterhin können Allele des glyA-Gens verwendet werden, die sich aus der Degeneriertheit des genetischen Codes oder durch funktionsneutrale Sinnmutationen (sense mutations) ergeben.

Zur Erzielung einer Abschwächung können entweder die Expression des glyA-Gens oder die katalytischen Eigenschaften des Genproduktes herabgesetzt oder ausgeschaltet werden. Gegebenenfalls werden beide Maßnahmen kombiniert.

Die Genexpression kann durch geeignete Kulturführung oder durch genetische Veränderung (Mutation) der Signalstrukturen der Genexpression verringert werden. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Pätek et al. (Microbiology 142: 1297 (1996)) und in bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung bzw. Herabsetzung der katalytischen Eigenschaften von Enzymproteinen führen, sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülich,Jül-2906, ISSN09442952, Jülich, Deutschland, 1994) genannt. Zusammenfassende Darstellungen können bekannten Lehrbüchern der Genetik und Molekularbiologie wie z. B. dem von Hagemann ("Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen (missense mutations) oder Nichtsinnmutationen (nonsense mutations) gesprochen. Insertionen oder Deletionen von mindestens einem Basenpaar in einem Gen führen zu Rasterverschiebungsmutationen (frame shift mutations), in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

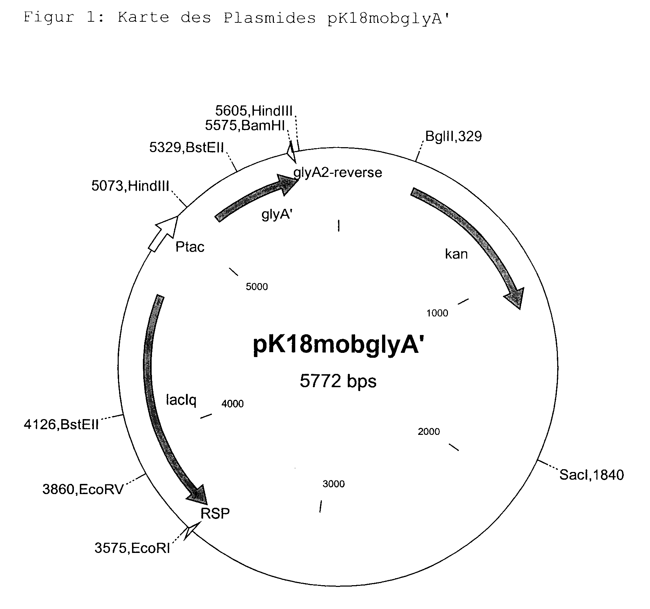

Beispielhaft wurde das glyA-Gen durch Entfernung des natürlichen Promotors und Vorschaltung eines stromaufwärtsgelegenen, regulierbaren Kontrollelementes abgeschwächt. Als Kontrollelement wurde das lacI-tac-System verwendet. Um den Einbau des lacI-tac-Systems stromaufwärts des chromosomalen glyA-Gens erzielen zu können, wurde das Integrationsplasmides pKl8mobglyA' (Figur 1) hergestellt. Das Plasmid pKl8mobglyA' enthält den tac-Promotor (Amann et al., Gene 25: 167-178 (1983); De Boer et al., Proceedings of the National Academy of Sciences of the United States of America USA 80: 21-25 (1983)) und direkt stromabwärts des tac-Promotors eine 5'-terminale Sequenz des glyA-Gens dargestellt in SEQ ID No 1. Das Plasmid enthält weiterhin das für den Lac-Inhibitor codierende lacI-Gen (Farabaugh, Nature 274: 765-769 (1978); Stark et al., Gene 51: 255-267 (1987)). Die Sequenz der lacI-tac-5'glyA Einheit ist in SEQ ID No 2 dargestellt. Plasmid pKl8mobglyA' ist in Escherichia coli nicht aber in Corynebacterium glutamicum replizierbar. Nach Transformation und homologer Rekombination mittels eines Integration bewirkenden "cross over"-Ereignisses erhält man eine intakte Kopie des glyA-Gens, dessen Expression durch das stromaufwärts gelegene lacI-tac-Kontrollelement kontrolliert bzw. reguliert werden kann und eine am 3'-Terminus verstümmelte (truncated), inaktive Kopie des glyA-Gens einschließlich des natürlichen Promotors. Die Sequenz der lacI-tac-glyA-Einheit ist in SEQ ID No 3 dargestellt. SEQ ID No 4 zeigt die bekannte Aminosäuresequenz des glyA-Genproduktes. Durch Zugabe geeigneter Konzentrationen des Lactoseanalogons Isopropylthiogalaktosid (Fürste et al., Gene 48: 119-131 (1986)) kann die Expression des glyA-Gens kontrolliert bzw. der zelluläre Gehalt an Serinhydroxymethyltransferase abgeschwächt bzw. eingestellt werden.

Weitere Anleitungen und Erläuterungen zur Integrationsmutagenese findet man beispielsweise bei Schwarzer und Pühler (Bio/Technology 9,84-87 (1991)), Peters-Wendisch et al. (Microbiology 144, 915-927 (1998)) oder Fitzpatrick et al. (Applied Microbiology Biotechnology 42, 575-580 (1994)).

Ein Beispiel für einen Aminosäure produzierenden Stamm coryneformer Bakterien mit abgeschwächtem glyA-Gen ist der Threoninproduzent Corynebacterium glutamicum DM368-2::pK18mobglyA'.

Zusätzlich kann es für die Produktion von Aminosäuren, vorteilhaft sein, zusätzlich zur Abschwächung des glyA-Gens, eines oder mehrere Enzyme des jeweiligen Biosyntheseweges, der Glykolyse, der Anaplerotik, des Zitronensäure-Zyklus oder des Aminosäure-Exports zu verstärken.

So kann beispielsweise für die Herstellung von L-Threonin
- gleichzeitig das für die Homoserin-Dehydrogenase kodierende hom-Gen (Peoples et al., Molecular Microbiology 2, 63-72 (1988)) oder das für eine "feed back resistente" Homoserin-Dehydrogenase kodierende hom^{dr}-Allel (Archer et al., Gene 107, 53-59 (1991)) und/oder
- gleichzeitig das für die Glyceraldehyd-3-Phosphat Dehydrogenase kodierende gap-Gen (Eikmanns et al., Journal of Bacteriology 174: 6076-6086 (1992)), oder
- gleichzeitig das für die Pyruvat Carboxylase codierende pyc-Gen (Peters-Wendisch et al., Microbiology 144: 915-927 (1998)), oder
- gleichzeitig das für die Malat:Chinon Oxidoreduktase kodierende mqo-Gen (Molenaar et al., European Journal of Biochemistry 254, 395-403 (1998)), oder
- gleichzeitig das für den Threonin-Export kodierende thrE-Gen (DE 199 41 478.5; DSM 12840)
überexprimiert werden.

Weiterhin kann es für die Produktion von Aminosäuren vorteilhaft sein, neben dem glyA-Gen gleichzeitig
- das für die Phosphoenolpyruvat-Carboxykinase codierende pck-Gen (DE 199 50 409.1; DSM 13047) und/oder
- das für die Pyruvat-Oxidase kodierende poxB-Gen (DE 199 51 975.7; DSM 13114)
abzuschwächen.

Schließlich kann es für die Produktion von Aminosäuren vorteilhaft sein, neben der Abschwächung des glyA-Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnußöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoffhaltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

Folgender Mikroorganismus wurde bei der Deutschen Sammlung für Mikroorganismen und Zellkulturen (DSMZ, Braunschweig, Deutschland) gemäß Budapester Vertrag hinterlegt:
- Escherichia coli Stamm DH5αmcr/pK18mobglyA' als DSM 13170

### Beispiele

Die vorliegende Erfindung wird im folgenden anhand von Ausführungsbeispielen näher erläutert.

Die Isolierung von Plasmid-DNA aus Escherichia coli sowie alle Techniken zur Restriktion, Klenow- und alkalische Phosphatasebehandlung wurden nach Sambrook et al. (Molecular cloning. A laboratory manual (1989) Cold Spring Harbour Laboratory Press) durchgeführt. Die Transformation von Escherichia coli wurde, wenn nicht anders beschrieben, nach Chung et al. (Proceedings of the National Academy of Sciences of the United States of America USA (1989) 86: 2172-2175) durchgeführt.

### Beispiel 1

### Klonierung und Sequenzierung des Gens glyA aus Corynebacterium glutamicum ATCC13032

Das Gen glyA wurde in den E. coli Klonierungsvektor pUC18 (Norrander et al., Gene (1983) 26: 101-106, Roche Diagnostics, Mannheim, Deutschland) kloniert. Die Klonierung wurde in zwei Schritten durchgeführt. Zunächst wurde durch eine Polymerasekettenreaktion (PCR) das Gen aus Corynebacterium glutamicum ATCC13032 mittels folgender aus der japanischen Offenlegungsschrift JP-A-08107788 abgeleiteter Oligonukleotid-Primer amplifiziert.

glyA1-forward:

5'-GCT TGC AGC GTT TTG CTC TGC C-3'

glyA1-reverse:

5'-ACC CGT AAC CTC TTC CAC ATA GG-3'

Die PCR-Reaktion wurde in 30 Zyklen in Gegenwart von 200 uM Deoxynukleotid-triphosphaten (dATP, dCTP, dGTP, dTTP), je 1 uM des entsprechenden Oligonukleotids, 100 ng chromosomaler DNA von Corynebacterium glutamicum ATCC13032, 1/10 Volumen 10-fach Reaktionspuffer und 2,6 Einheiten einer hitzestabilen Taq-/Pwo-DNA-Polymerase-Mischung (Expand High Fidelity PCR System der Firma Roche Diagnostics, Mannheim, Deutschland) in einem Thermocycler (PTC-100, MJ Research, Inc., Watertown, USA) unter folgenden Bedingungen durchgeführt: 94°C für 30 Sekunden, 64°C für 1 Minute und 68°C für 3 Minuten.

Das amplifizierte etwa 1,7 kb große Fragment wurde dann im folgenden mit Hilfe des SureClone Ligation Kit (Amersham Pharmacia Biotech, Uppsala, Schweden) nach Angaben des Herstellers in die SmaI-Schnittstelle des Vektors pUC18 ligiert. Mit dem gesamten Ligationsansatz wurde der E. coli Stamm DH5amcr (Grant et al., Proceedings of the National Academy of Sciences of the United States of America USA (1990) 87: 4645-4649) transformiert. Transformanten wurden anhand ihrer Carbenicillinresistenz auf 50 µg/mL Carbenicillin enthaltenden LB-Agarplatten identifiziert. Aus 7 der Transformanten wurden die Plasmide präpariert und durch Restriktionsanalyse auf das Vorhandensein des 1,7 kb PCR-Fragments als Insert überprüft. Das so entstandene rekombinante Plasmid wird im folgenden mit pUC18glyA bezeichnet.

Die Nukleotidsequenz des 1,7 kb PCR-Fragments in Plasmid pUC18glyA wurde nach der Dideoxy-Kettenabbruchmethode von Sanger et al. bestimmt (Proceedings of the National Academy of Sciences of the United States of America USA (1977) 74: 5463-5467). Hierzu wurde das vollständige Insert von pUC18glyA unter Zuhilfenahme der nachfolgenden Primer sequenziert.

Universalprimer: 5'-GTA AAA CGA CGG CCA GT-3'

Reverseprimer: 5'-GGA AAC AGC TAT GAC CAT G-3'

Die erhaltenen Nukleotidsequenzen wurde mit dem Programmpaket Lasergene (Biocomputing Software for Windows, DNASTAR, Madison, USA) analysiert. Die Analyse ergab die Identifizierung eines offenen Leserasters von 1302 bp Länge. Das entsprechende Gen wurde als glyA-Gen bezeichnet. Das dazugehörige Genprodukt umfasst 434 Aminosäuren und ist als SEQ ID No 4 wiedergegeben.

### Beispiel 2

### Konstruktion eines Vektors zur reduzierten Expression von glyA

Aus dem unter Beispiel 1 beschriebenen Plasmid pUC18glyA wurde mit den Restriktionsenzymen EcoRI und TfiI ein 1418 bp grosses DNA-Fragment ausgeschnitten, welches das glyA-Gen ohne eigenen Promotorbereich enthält. Die 5'- und 3'-Enden dieses Fragments wurden mit Klenow-Enzym behandelt. Das resultierende DNA-Fragment wurde so in den zuvor mit BamHI linearisierten, mit Klenow-Enzym behandelten und dephosphorylierten Vektor pVWEx2 (Wendisch, "Physiologische und NMR-spektroskopische Untersuchungen zur in vivo-Aktivität zentraler Stoffwechselwege im Wildstamm und in rekombinanten Stämmen von Corynebacterium glutamicum", Berichte des Forschungszentrums Jülich, Jül-3397, ISSN09442952, Jülich, Deutschland, 1997) ligiert, daß das glyA-Gen in gleicher Orientierung unmittelbar hinter dem mit Isopropyl-β-D-thiogalaktosid (IPTG) induzierbaren tac-Promotor des Vektors liegt. Mit dem gesamten Ligationsansatz wurde der E. coli Stamm DH5amcr (Grant et al., Proceedings of the National Academy of Sciences of the United States of America USA (1990) 87: 4645-4649) transformiert. Transformanten wurden anhand ihrer Tetracyclinresistenz auf 15 µg/mL Tetracyclin enthaltenden LB-Agarplatten identifiziert. Aus 12 Transformanten wurden die Plasmide präpariert und durch Restriktionsanalyse auf das Vorhandensein des 1418 bp Fragments als Insert in der richtigen Orientierung bezüglich des tac-Promotors überprüft. Das auf diese Weise entstandene rekombinante Plasmid wird im folgenden mit pVWEx2glyA bezeichnet.

Aus dem Plasmid pVWEx2glyA wurde dann durch eine Polymerasekettenreaktion (PCR) mittels folgender Oligonukleotid-Primer ein DNA-Fragment amplifiziert, welches lacI, das Gen für den Repressor des tac-Promotors, den tac-Promotor und die ersten 438 bp des klonierten glyA-Gens von Corynebacterium glutamicum enthält.
glyA2-forward (mit angefügter, durch Unterstreichung gekennzeichneter EcoRI-Erkennungssequenz): 5'-CCG GAA TTC TCA CTG CCC GCT TTC CAG TC-3' glyA2-reverse (mit angefügter, durch Unterstreichung gekennzeichneter BamHI-Erkennungssequenz):

5'-CGG GAT CCC AGC TTT CCG GAG AAG TTC AAC-3'

Die PCR-Reaktion wurde in 30 Zyklen in Gegenwart von 200 µM Deoxynukleotid-triphosphaten (dATP, dCTP, dGTP, dTTP), je 1 uM des entsprechenden Oligonukleotids, 100 ng Plasmid-DNA von pVWEx2glyA, 1/10 Volumen 10-fach Reaktionspuffer und 2,6 Einheiten einer hitzestabilen Taq-/Pwo-DNA-Polymerase-Mischung (Expand High Fidelity PCR System der Firma Roche Diagnostics, Mannheim, Deutschland) in einem Thermocycler (PTC-100, MJ Research, Inc., Watertown, USA) unter folgenden Bedingungen durchgeführt: 94°C für 30 Sekunden, 58°C für 30 Sekunden und 72°C für 2 Minuten.

Das amplifizierte etwa 2,0 kb große Fragment wurde im folgenden mit EcoRI und BamHI verdaut, mit Hilfe des NucleoSpin Extract 2 in 1 Kit der Firma Macherey-Nagel (Düren, Deutschland) nach Angaben des Herstellers isoliert und dann in den ebenfalls mit EcoRI und BamHI geschnittenen und dephosphorylierten Vektor pKl8mob (Schäfer et al., Gene (1994) 145: 69-73) ligiert. Mit dem gesamten Ligationsansatz wurde der E. coli Stamm DH5αmcr (Grant et al., Proceedings of the National Academy of Sciences of the United States of America USA (1990) 87: 4645-4649) transformiert. Transformanten wurden anhand ihrer Kanamycinresistenz auf 50 ug/mL Kanamycin enthaltenden LB-Agarplatten identifiziert. Aus 12 der Transformanten wurden die Plasmide präpariert und durch Restriktionsanalyse auf das Vorhandensein des 2,0 kb PCR-Fragments als Insert überprüft. Das so entstandene rekombinante Plasmid wird im folgenden mit pKl8mobglyA' bezeichnet (siehe Figur 1).

### Beispiel 3

### Konstruktion des Stammes Corynebacterium glutamicum ATCC13032::pK18mobglyA' mit reduzierter, regulierbarer glyA-Expression

Mittels Elektroporation (Haynes et al., FEMS Microbiology Letters (1989) 61: 329-334) wurden der Leervektor pZl (Menkel et al., Applied and Environmental Microbiology (1989) 64: 549-554) und das in Beispiel 2 beschriebene Plasmid pK18mobglyA' in den Wildtypstamm Corynebacterium glutamicum ATCC13032 (Abe et al., Journal of General and Applied Microbiology (1967) 13: 279-301) eingebracht.

Nach Transformation mit pZl wurden die Transformanten anhand ihrer Kanamycinresistenz auf 15 ug/mL Kanamycin enthaltenden LBHIS-Agarplatten identifiziert (Liebl et al., FEMS Microbiology Letters (1989) 65: 299-304). Aus 3 der Transformanten wurden die Plasmide präpariert und durch Restriktionsanalyse auf das Vorhandensein des pZl-Leervektors überprüft. Auf diese Weise entstand der Kontrollstamm Corynebacterium glutamicum ATCC13032/pZl.

Nach Transformation mit pKl8mobglyA' mußte das Plasmid über homologe Rekombination des klonierten 5'-Endes von glyA in das Chromosom von Corynebacterium glutamicum ATCC13032 integrieren. Die erhaltenen Kanamycin resistenten Klone wurden auf 15 µg/mL Kanamycin und 1 mM Isopropyl-β-D-thiogalaktosid (IPTG) enthaltenden LBHIS-Agarplatten identifiziert (Liebl et al., FEMS Microbiology Letters (1989) 65: 299-304). Die korrekte Integration von pKl8mobglyA' im Chromosom wurde in 2 erhaltenen Integrationsmutanten durch eine Polymerasekettenreaktion (PCR) mittels folgender Oligonukleotid-Primer überprüft.

Reverseprimer (RSP):
5'-GGA AAC AGC TAT GAC CAT G-3'

glyA2-reverse:
5'-CGG GAT CCC AGC TTT CCG GAG AAG TTC AAC-3'

Die PCR-Reaktion wurde in 30 Zyklen in Gegenwart von 200 uM Deoxynukleotid-triphosphaten (dATP, dCTP, dGTP, dTTP), je 1 uM des entsprechenden Oligonukleotids, 100 ng chromosomaler DNA von Corynebacterium glutamicum ATCC13032::pK18mobglyA', 1/10 Volumen 10-fach Reaktionspuffer und 2,6 Einheiten einer hitzestabilen Taq-/Pwo-DNA-Polymerase-Mischung (Expand High Fidelity PCR System der Firma Roche Diagnostics, Mannheim, Deutschland) in einem Thermocycler (PTC-100, MJ Research, Inc., Watertown, USA) unter folgenden Bedingungen durchgeführt: 94°C für 30 Sekunden, 48°C für 30 Sekunden und 72°C für 2 Minuten. Auf diese Weise entstand der Stamm Corynebacterium glutamicum ATCC13032::pK18mobglyA', in dem das glyA-Gen unter der Kontrolle des mit Isopropyl-β-D-thiogalaktosid (IPTG) induzierbaren tac-Promotors vorliegt.

### Beispiel 4

### Bestimmung der durch das glyA-Gen kodierten Serinhydroxymethyltransferase-Aktivität in dem Stamm Corynebacterium glutamicum ATCC13032::pK18mobglyA'

Zur Gewinnung der Rohextrakte für die Bestimmung der durch glyA kodierten Serinhydroxymethyltransferase-Aktivität wurden die in Beispiel 3 beschriebenen Stämme C. glutamicum ATCC13032/pZl und C. glutamicum ATCC13032::pK18mobglyA' in 100 mL Brain Heart Infusion-Medium (Difco Laboratories, Detroit, USA) mit 25 ug Kanamycin/mL und 100 µM Isopropyl-β-D-thiogalaktosid (IPTG) für 14 Stunden bei 30°C vorkultiviert. Anschließend wurden die Zellen einmal mit 0,9%(w/v) Natriumchloridlösung gewaschen und mit dieser Suspension je 100 mL CgXII-Medium so angeimpft, daß die OD600 (optische Dichte bei 600 nm) 0,5 betrug. Das Medium war identisch mit dem bei Keilhauer et al. beschriebenen Medium (Journal of Bacteriology (1993) 175: 5593-5603), enthielt aber zusätzlich 25 ug Kanamycin/mL und 0, 10 oder 100 µM Isopropyl-β-D-thiogalaktosid (IPTG). Die Zusammensetzung des von Keilhauer et al. beschriebenen Mediums ist in Tabelle 1 dargestellt.

**Tabelle 1**

| Zusammensetzung des Mediums CGXII | |
|---|---|
| Komponente | Konzentration |
| (NH₄)₂SO₄ | 20 g/L |
| Harnstoff | 5 g/L |
| KH₂PO₄ | 1 g/L |
| K₂HPO₄ | 1 g/L |
| MgSO₄ x 7 H₂O | 0,25 g/L |
| 3-Morpholinopropansulfonsäure | 42 g/L |
| CaCl2 | 10 mg/L |
| FeSO₄ x 7 H₂O | 10 mg/L |
| MnSO₄ x H₂O | 10 mg/L |
| ZnSO₄ x 7H₂O | 1 mg/L |
| CuSO₄ | 0,2 mg/L |
| NiCl₂ x 6 H20 | 0,02 mg/L |
| Biotin | 0,2 mg/L |
| Glukose | 40 g/L |
| Protokatechusäure | 30 mg/L |

Die Kultivierung beider Stämme wurde bei 30°C durchgeführt. Nach 10 Stunden wurden die Zellen einmal mit 50 mM 4-(2-Hydroxyethyl)-1-piperazinethansulfonsäure/Natriumhydroxid-Puffer (pH 7,0) gewaschen, abzentrifugiert (10 Minuten bei 5000 Umdrehungen pro Minute mit einer Minifuge RF der Firma Heraeus, Osterode, Deutschland) und in 200 mM 4-(2-Hydroxyethyl)-1-piperazinethansulfonsäure/Natriumhydroxid-Puffer (pH 7,0) resuspendiert, so daß das Endvolumen 5 mL betrug. Zu dieser Zellsuspension wurden 50 µL 2 mM Pyridoxal-5-phosphat-Lösung und 50 uL 100 mM Dithiothreitol-Lösung gegeben und die Zellen aufgeschlossen. Der Zellaufschluß erfolgte bei 0°C durch einen Ultraschalldesintegrator (Branson Sonifier W-250, Branson Sonic Power Co, Danbury, USA; Beschalldauer 6 Minuten, Pulslänge 100%, Beschallintensität 2,5). Nach der Ultraschallbehandlung wurden die Zelltrümmer durch Zentrifugation (30 Minuten bei 4°C und 13000 Umdrehungen pro Minute in einer kühlbaren Zentrifuge Sigma 202 MK der Firma Sigma-Aldrich, Deisenhofen, Deutschland) abgetrennt. Der Überstand wurde als zellfreier Rohextrakt direkt zur Bestimmung der Enzymaktivität eingesetzt.

Die Proteinbestimmung in den zellfreien Rohextrakten wurde photometrisch nach Bensadoun und Weinstein (Analytical Biochemistry (1976) 70: 241-250) durchgeführt. Der Proteingehalt wurde dabei über eine mit Rinderserumalbumin als Standard erstellte Eichkurve ermittelt.

Zur Bestimmung der Aktivität der Serinhydroxymethyltransferase in den zellfreien Rohextrakten wurde ein diskontinuierlicher Enzymtest verwendet, bei dem das aus dem Substrat Threonin gebildete Glycin quantifiziert wurde. Die Reaktionsansätze wurden in der folgenden Zusammensetzung (modifiziert nach Scrimgeour und Huennekens, Methods in Enzymology (1962) Vol. V: 838-843, Academic Press) 15 Minuten bei 37°C inkubiert: 20 mM Threonin, 200 uM Pyridoxal-5-phosphat, 900 µM Tetrahydrofolat, 100 mM 4-(2-Hydroxyethyl)-1-piperazinethansulfonsäure/Natriumhydroxid-Puffer (pH 7,0) und 1,0-1,5 mg Protein (aus Rohextrakt) in einem Endvolumen von 1 mL. Die Reaktion wurde durch Zugabe von 0,25 Volumen 25% (w/v) Trichloressigsäurelösung abgestoppt, die Ansätze 15 Minuten bei 0°C inkubiert und das denaturierte Protein abzentrifugiert (15 Minuten bei 4°C und 13000 Umdrehungen pro Minute in einer kühlbaren Zentrifuge Sigma 202 MK der Firma Sigma-Aldrich, Deisenhofen, Deutschland). Die quantitative Bestimmung des im Enzymtest gebildeten Glycins aus dem Überstand erfolgte mittels reversed phase HPLC (Lindroth et al., Analytical Chemistry (1979) 51: 1167-1174). Es wurde ein HPLC-Gerät der Serie HP1100 (Hewlett-Packard, Waldbronn, Deutschland) mit angeschlossenem Fluoreszenzdetektor (G1321A) verwendet; die Systemsteuerung und die Auswertung der Daten erfolgte mit einer HP-Chem-Station (Hewlett-Packard). 1 pL der zu analysierenden Aminosäurelösung wurde in einer automatischen Vorsäulenderivatisierung mit 20 uL ortho-Phthalaldehyd/2-Mercaptoethanol-Fertigreagenz (Pierce Europe BV, Oud-Beijerland, Niederlande) gemischt. Die dabei entstehenden fluoreszierenden, thiosubstituierten Isoindole (Jones et al., Journal of Chromatography (1983) 266: 471-482) wurden über eine kombinierte Vorsäule (40x4 mm Hypersil ODS 5) und Hauptsäule (Hypersil ODS 5, beide Säulen von der Firma CS-Chromatographie Service GmbH, Langerwehe, Deutschland) mit einem Gradientenprogramm mit zunehmend unpolarer Phase (Methanol) aufgetrennt. Das polare Eluenz war Natriumacetat (0,1 Molar, pH 7,2); die Flußrate betrug 0,8 mL pro Minute. Die Fluoreszenzdetektion der derivatisierten Aminosäuren erfolgte bei einer Anregungswellenlänge von 230 nm und einer Emissionswellenlänge von 450 nm. Die Glycinkonzentrationen wurden über einen Vergleich mit einem externen Standard und Asparagin als zusätzlichem internen Standard berechnet.

Die Ergebnisse des Enzymtests mit Threonin als Substrat sind in Tabelle 2 aufgeführt.

**Tabelle 2:**

| Stamm | IPTG-Konzentration (µM) | Serinhydroxymethyltransferase -Aktivität (nmol Glycin/Minute/mg Protein) |
|---|---|---|
| ATCC13032/pZl | 0 | 0,9 |
| ATCC13032::pK18mobglyA' | 0 | 0,3 |
| | 10 | 0,7 |
| | 100 | 1,6 |

### Beispiel 5

### Konstruktion des Stammes Brevibacterium flavum DM368-2::pK18mobglyA' mit reduzierter, regulierbarer glyA-Expression

Mittels Elektroporation (Haynes et al., FEMS Microbiology Letters (1989) 61: 329-334) wurden der Leervektor pZl (Menkel et al., Applied and Environmental Microbiology (1989) 64: 549-554) und das in Beispiel 2 beschriebene Plasmid pK18mobglyA' in den threoninbildenden Stamm Brevibacterium flavum DM368-2 eingebracht. Der Stamm DM368-2 ist in der EP-B-0 385 940 beschrieben und als DSM5399 hinterlegt.

Nach Transformation mit pZl wurden die Transformanten anhand ihrer Kanamycinresistenz auf 15 ug/mL Kanamycin enthaltenden LBHIS-Agarplatten identifiziert (Liebl et al., FEMS Microbiology Letters (1989) 65: 299-304). Aus 3 der Transformanten wurden die Plasmide präpariert und durch Restriktionsanalyse auf das Vorhandensein des pZ1-Leervektors überprüft. Auf diese Weise entstand der Kontrollstamm Brevibacterium flavum DM368-2/pZl.

Nach Transformation mit pK18mobglyA' mußte das Plasmid über homologe Rekombination des klonierten 5'-Endes von glyA in das Chromosom von Brevibacterium flavum DM368-2 integrieren. Die erhaltenen Kanamycin resistenten Klone wurden auf 15 ug/mL Kanamycin und 1 mM Isopropyl-β-D-thiogalaktosid (IPTG) enthaltenden LBHIS-Agarplatten identifiziert (Liebl et al., FEMS Microbiology Letters (1989) 65: 299-304). Die korrekte Integration von pK18mobglyA' im Chromosom wurde in 4 erhaltenen Integrationsmutanten durch eine Polymerasekettenreaktion (PCR), wie bereits in Beispiel 3 beschrieben, mit 100 ng chromosomaler DNA von Brevibacterium flavum DM368-2::pK18mobglyA' als Template überprüft. Auf diese Weise entstand der Stamm Brevibacterium flavum DM368-2::pK18mobglyA', in dem das glyA-Gen unter der Kontrolle des mit Isopropyl-β-D-thiogalaktosid (IPTG) induzierbaren tac-Promotors vorliegt.

### Beispiel 6

### Bestimmung der durch glyA kodierten Serinhydroxymethyltransferase-Aktivität in dem Stamm Brevibacterium flavum DM368-2::pK18mobglyA'

Die Gewinnung der Rohextrakte für die Bestimmung der durch glyA kodierten Serinhydroxymethyltransferase-Aktivität in den in Beispiel 5 beschriebenen Stämmen B. flavum DM368-2/pZl und B. flavum DM368-2::pK18mobglyA' wurde, wie bereits in Beispiel 4 beschrieben, durchgeführt. Die Proteinbestimmung in den erhaltenen zellfreien Rohextrakten und der diskontinuierliche Enzymtest, bei dem das aus dem Substrat Threonin gebildete Glycin quantifiziert wird, wurde ebenfalls wie in Beispiel 4 beschrieben durchgeführt.

Die Ergebnisse dieses Enzymtests mit Threonin als Substrat sind in Tabelle 3 aufgeführt.

**Tabelle 3:**

| Stamm | IPTG-Konzentration (µM) | Serinhydroxymethyltransferase-Aktivität (nmol Glycin/Minute/mg Protein) |
|---|---|---|
| DM368-2/pZl | 0 | 1,6 |
| DM368-2::pK18mobglyA' | 0 | <0, 1 |
| | 10 | 0,8 |
| | 100 | 1,7 |

### Beispiel 7

### Herstellung von L-Threonin mit Brevibacterium flavum

Zur Untersuchung ihrer Threoninbildung wurden die in Beispiel 5 beschriebenen Stämme B. flavum DM368-2/pZl und DM368-2::pKl8mobglyA' in 100 mL Brain Heart Infusion-Medium (Difco Laboratories, Detroit, USA) mit 25 µg Kanamycin/mL und 100 uM Isopropyl-β-D-thiogalaktosid (IPTG) für 14 Stunden bei 30°C vorkultiviert. Anschließend wurden die Zellen einmal mit 0,9%(w/v) Natriumchloridlösung gewaschen und mit dieser Suspension je 60 mL CgXII-Medium so angeimpft, daß die OD₆₀₀ (optische Dichte bei 600 nm) 0,5 betrug. Das Medium war identisch mit dem bei Keilhauer et al. beschriebenen Medium (Journal of Bacteriology (1993) 175: 5593-5603), enthielt aber zusätzlich 25 µg Kanamycin/mL und 0, 10 oder 100 uM Isopropyl-β-D-thiogalaktosid (IPTG).

Die Kultivierung beider Stämme wurde bei 30°C über einen Zeitraum von 72 Stunden durchgeführt. Nach 48 und 72 Stunden wurden jeweils Proben genommen und die Zellen kurz abzentrifugiert (5 Minuten bei 13000 Umdrehungen pro Minute mit einer Biofuge pico der Firma Heraeus, Osterode, Deutschland).

Die quantitative Bestimmung der extrazellulären Aminosäurekonzentrationen aus dem Kulturüberstand erfolgte wie bereits in Beispiel 4 beschrieben mittels reversed phase HPLC (Lindroth et al., Analytical chemistry (1979) 51: 1167-1174). Die Threoninkonzentrationen wurden über einen Vergleich mit einem externen Standard und Asparagin als zusätzlichem internen Standard berechnet.

Die Ergebnisse sind in Tabelle 4 aufgeführt.

**Tabelle 4:**

| Stamm | IPTG-Konzentration | L-Threonin (g/1) | |
|---|---|---|---|
| | µM | 48 Stunden | 72 Stunden |
| DM368-2/pZl | 0 | 1,27 | 1,32 |
| DM368-2::pK18mobglyA' | 10 | 1,32 | 1,44 |
| | 0 | 1,41 | 1,60 |

Folgende Figuren sind beigefügt:
Figur 1: Karte des Plasmids pKl8mobglyA'. Längenangaben sind als ca.-Werte aufzufassen.

Die verwendeten Abkürzungen und Bezeichnungen haben folgende Bedeutung.
- BamHI: Restriktionsendonuklease aus Bacillus amyloliquefaciens
- BglII: Restriktionsendonuklease aus Bacillus globigii
- BstEII: Restriktionsendonuklease aus Bacillus stearothermophilus
- EcoRI: Restriktionsendonuklease aus Escherichia coli
- EcoRV: Restriktionsendonuklease aus Escherichia coli
- HindIII: Restriktionsendonuklease aus Haemophilus influenzae
- SacI: Restriktionsendonuklease aus Streptomyces achromogenes
- kan: Kanamycinresistenzgen
- lacl^{q}: Gen für den Repressor des tac-Promotors Ptac
- Ptac: tac-Promotor
- glyA': 5'-Teil des Serinhydroxymethyltransferasegens
- glyA2-reverse: Primer zur Überprüfung einer Integration
- RSP: Reverse Standardprimer zur Überprüfung einer Integration

## Patentansprüche

1. Verfahren zur Herstellung von L-Aminosäuren
**dadurch gekennzeichnet,**
daß man folgende Schritte durchführt,
a) Fermentation der die gewünschte L-Aminosäure produzierenden coryneformen Bakterien, in denen man zumindest das glyA-Gen abschwächt,
b) Anreicherung des gewünschten Produkts im Medium oder in den Zellen der Bakterien, und
c) Isolieren der L-Aminosäure.

2. Verfahren gemäß Anspruch 1,
**dadurch gekennzeichnet,**
daß man Bakterien einsetzt, in denen man zusätzlich weitere Gene des Biosyntheseweges der gewünschten L-Aminosäure verstärkt.

3. Verfahren gemäß Anspruch 1,
**dadurch gekennzeichnet,**
daß man Bakterien einsetzt, in denen die Stoffwechselwege zumindest teilweise ausgeschaltet sind, die die Bildung der gewünschten L-Aminosäure verringern.

4. Verfahren gemäß Anspruch 1,
**dadurch gekennzeichnet,**
daß man die Expression des Polynukleotids, das für das glyA-Gen codiert, verringert.

5. Verfahren gemäß Anspruch 1,
**dadurch gekennzeichnet,**
daß man die katalytischen Eigenschaften des Polypeptids (Enzymproteins) herabsetzt, für das das Polynukleotid glyA codiert.

6. Verfahren gemäß Anspruch 1,
**dadurch gekennzeichnet,**
daß man zur Erzielung der Abschwächung das Verfahren der Integrationsmutagenese mittels des Vektors pKl8mobglyA', dargestellt in Figur 1 und hinterlegt in E.coli als DSM 13170, verwendet.

7. Verfahren gemäß Anspruch 1,
**dadurch gekennzeichnet,**
daß man für die Herstellung von L-Threonin Bakterien fermentiert, in denen man gleichzeitig eines oder mehrere der Gene, ausgewählt aus der Gruppe
7.1 das für die Homoserin-Dehydrogenase kodierende hom-Gen
7.2 das für die Glyceraldehyd-3-Phosphat Dehydrogenase kodierende gap-Gen,
7.3 das für die Pyruvat-Carboxylase kodierende pyc-Gen,
7.4 das für die Malat:Chinon Oxidoreduktase kodierende mqo-Gen,
7.5 das für den Threonin-Export kodierende thrE-Gen,
gleichzeitig überexprimiert oder amplifiziert.

8. Verfahren gemäß Anspruch 1,
**dadurch gekennzeichnet,**
daß man für die Herstellung von L-Threonin Bakterien fermentiert, in denen man gleichzeitig eines oder mehrere der Gene, ausgewählt aus der Gruppe
8.1 das für die Phosphoenolpyruvat-Carboxykinase codierende pck-Gen,
8.2 das für die Pyruvat-Oxidase codierende poxB-Gen
abschwächt.

9. Verfahren gemäß einem oder mehreren der vorhergehenden Ansprüche,
**dadurch gekennzeichnet,**
daß man Mikroorganismen der Gattung Corynebacterium glutamicum einsetzt.

10. Coryneforme Bakterien, in denen man das glyA-Gen abschwächt.

11. Vektor pK18mobglyA', dargestellt in Figur 1 und hinterlegt in E.coli als DSM 13170.

12. Isoliertes Polynukleotid, enthaltend
(i) die Nukleotidsequenz der lacI-tac-5`glyA-Einheit, dargestellt in SEQ ID No. 2, oder
(ii) mindestens eine Sequenz, die der Sequenz (i) innerhalb des Bereichs der Degeneration des genetischen Codes entspricht, oder
(iii) mindestens eine Sequenz, die mit den zu den Sequenzen (i) oder (ii) komplementären Sequenzen hybridisiert, und gegebenenfalls
(iv) funktionsneutrale Sinnmutanten in (i).

13. Isoliertes Polynukleotid, enthaltend
(i) die Nukleotidsequenz der lacI-tac-glyA-Einheit, dargestellt in SEQ ID No. 3, oder
(ii) mindestens eine Sequenz, die der Sequenz (i) innerhalb des Bereichs der Degeneration des genetischen Codes entspricht, oder
(iii) mindestens eine Sequenz, die mit den zu den Sequenzen (i) oder (ii) komplementären Sequenzen hybridisiert, und gegebenenfalls
(iv) funktionsneutrale Sinnmutanten in (i).
